# EUROPEAN PATENT APPLICATION

(11) **EP 4 286 398 A2**
(43) Date of publication of application: **06.12.2023**
(21) Application number: 22746250.4
(22) Date of filing: 27.01.2022
(51) Int. Cl.: C07K 1/12, C07K 14/245, G01N 33/68

(54) **METHOD FOR SEPARATING GRAM-NEGATIVE BACTERIUM-DERIVED INTACT PROTEIN**

(30) Priority: 27.01.2021 KR 20210011737
(71) Applicant: Seegene Medical Foundation, Seoul 04805 (KR)
(72) Inventor: CHUN, Jong Kee, Seoul 04805 (KR); BAEK, Je Hyun, Gwacheon-si, Gyeonggi-do 13827 (KR); YANG, Won Suk, Bucheon-si, Gyeonggi-do 14774 (KR); LEE, Saeyoung, Suwon-si, Gyeonggi-do 16361 (KR); CHEON, Dong Huey, Seongnam-si, Gyeonggi-do 13528 (KR); JANG, Heejung, Seoul 02603 (KR); HWANG, Seohyun, Seoul 02752 (KR)
(74) Representative: Müller, Christian Stefan Gerd
(86) International application number: PCT/KR2022/001480
(87) International publication number: WO 2022/164224

(57) **Abstract**

The present invention relates to a method for eluting proteins from prokaryotes. The present invention enables easy break down of bacterial cell walls to obtain intact proteins without damage by a simple process of adding organic solvents including lower alcohols or nitrile derivatives; or applying osmotic stimulation to samples containing pathogenic bacteria or the like. In particular, the present invention may be usefully applied to rapid and accurate identification of periplasmic proteins of gram-negative bacteria without additional purification process.

## Description

### TECHNICAL FIELD

The present invention relates to a method for efficiently isolating intact proteins derived from gram-negative bacteria by lysing the bacteria with osmotic stimulation and/or organic solvents.

### BACKGROUND ART

Rapid and accurate detection of genes or proteins that could be indicators of specific pathogens are important not only for diagnosis of pathogenic infection but also for early establishment of treatment strategies through phenotypic analysis such as antibiotic resistance. Genetic diagnosis technology using real-time PCR has limitations in its application for rapid and accurate mass diagnosis since it requires complex processes including gene extraction and amplification, high-cost sample preprocessing, and prior information on the nucleotide sequence of the target gene.

On the other hand, mass spectrometry, such as MALDI-TOF, is an analyzing system with low-cost and high-efficiency relative to sequencing methods such as PCR, that can be an important means for rapid detection capable of completing from sample processing to identification of the strain after culturing within 10 minutes, and quickly identifying an unknown strain whose mass values match by comparing the measured mass data with a constructed database.

However, it is necessary to expose the marker protein present in the bacterial cell wall to the outside culture environment for mass spectrometry. For this, conventional microbial protein extraction methods have generally used a chaotropic agent, an ionic or nonionic surfactant to suppress hydrogen bonds in substances, or adopted ultrasonic disruption to dissolve or disrupt microorganisms. Since these methods affect the sensitivity of the mass spectrometer and generate lots of noise on the mass spectrometry spectrum, additional purification steps prior to analysis were needed. Accordingly, another disadvantage lies in that the pretreatment process before analysis takes a lot of time and cost, and requires expensive equipment.

Therefore, there is a need to develop more effective method of sample pretreatment for rapid and accurate diagnosis of infectious disease or analysis of the biological composition of a sample.

Throughout this application, various publications and patents are referred and citations are provided in parenthesis. The disclosure of these publications and patents in their entities are hereby incorporated by references into this application in order to fully describe this invention and the state of the art to which this invention pertains.

### TECHNICAL PROBLEM

The present inventors have made intensive studies to develop an effective method for eluting a prokaryotic protein capable of obtaining intact proteins while efficiently dissolving various prokaryotes including pathogenic bacteria. As results, the present inventors have discovered that, by disrupting the cell walls of prokaryotes through a simple process of contacting a sample containing prokaryotes with organic solvents comprising lower alcohols or nitrile derivatives, completely intact proteins may be obtained without physical, chemical or enzymatic damage thus may be usefully applied to protein identification for various purposes such as diagnosis or proteomic analysis.

Accordingly, it is an object of the present invention to provide a method for eluting a protein from a prokaryote.

It is another object of the present invention to provide a composition for eluting a protein from a prokaryote.

Other objects and advantages of the present invention will become more apparent from the following detailed description of the invention, claims and drawings.

### TECHNICAL SOLUTION

In one aspect of the present invention, there is provided a method for eluting a protein of a prokaryote comprising adding at least one organic solvent selected from the group consisting of C₁-C₄ alcohols and R-CN (wherein R is a straight or branched C₁-C₃ alkyl) to a biological sample comprising the prokaryote.

The present inventors have made intensive studies to develop an effective method for eluting a prokaryotic protein capable of obtaining intact proteins while efficiently dissolving various prokaryotes including pathogenic bacteria. As results, the present inventors have discovered that, by disrupting the cell walls of prokaryotes through a simple process of contacting a sample containing prokaryotes with organic solvents comprising lower alcohols or nitrile derivatives, completely intact proteins may be obtained without physical, chemical or enzymatic damage thus may be usefully applied to protein identification for various purposes such as diagnosis or proteomic analysis.

The term "prokaryote" as used herein refers to cellular organisms without a distinct nucleus surrounded by a nuclear membrane. Prokaryotes are classified into three types according to their appearance: rod-shaped bacillus, spherical coccus, and spiral-shaped spirillium, and are composed of an outer membrane, inner membrane or cytoplasmic membrane, nuclear region, ribosomes, storage granules and volutin. The outer membrane serves to support cells preserve their original form in the external environment, and the inner membrane or cytoplasmic membrane selectively transports ions or nutrients required from the outside and regulates rate of transport. Therefore, structural loss leads to cell lysis.

According to a concrete embodiment of the present invention, the prokaryote is gram-negative bacteria. The term "gram-negative bacteria" as used herein refers to bacteria that are stained red upon gram staining and have strong resistance to dyes and surfactants. The gram-negative bacteria to which the method of the present invention is applied may comprise *Escherichia coli, Citrobacter koseri, Enterobacter aerogenes, Escherichia albertii, Escherichia blattae, Escherichia fergusonii, Escherichia hermannii, Escherichia vulneris, Acinetobacter baumannii, Acinetobacter junii, Acinetobacter boissieri, Acinetobacter calcoaceticus, Acinetobacter haemolyticus, Acinetobacter nosocomialis, Acinetobacter schindleri, Acinetobacter ursingii, Klebsiella pneumoniae, Staphylococcus aureus, Streptococcus pneumoniae, Neisseria gonorrhoeae, Pseudomonas aeruginosa, Samonella typhimurium, Clostridium difficile, Shigella, Yersinia pestis, Haemophilus, Brucella, Legionella, Burkholderia cepacia, Vibrio* and *Stenotrophomonas maltophilia,* but are not limited thereto.

The term "protein" as used herein refers to linear molecules formed by binding of amino acid residues via peptide bonds. The protein to be eluted by the present invention may be a biological marker capable of identifying the presence or absence of prokaryotes in a sample (i.e. diagnosis of infection), their type, or their phenotypes such as antibiotic resistance.

The term "diagnosis" as used herein refers to determining a subject's susceptibility to a particular disease, determining whether a subject currently has a particular disease, or determining the prognosis of a subject suffering from a particular disease. For the purpose of the present invention, the term "diagnosis" as used herein generally refers to "determination of pathogenic infection".

In the present invention, "having antibiotic resistance" means that the microorganism can grow even in an environment where antibiotics for a specific pathogenic microorganism are present in a high concentration or an effective amount. Antibiotic resistance can be determined by detecting the presence of a digestive enzyme, a protein secreted by pathogenic microorganisms, that degrades the antibiotic to remove or reduce its activity. For example, beta-lactam antibiotics such as penicillin, cephalosporin, monobactam, and carbapenem, which inhibit cell wall synthesis, are neutralized by β-lactamase and may not inhibit pathogens that express such enzymes. Accordingly, microorganisms' antibiotic resistance can be determined depending on whether these proteins are detected. Hence, the term "resistant" is used synonymously with "tolerance" or "low therapeutic response".

According to a concrete embodiment, the protein to be eluted by the present invention may be periplasmic proteins of prokaryotes. The term "periplasmic protein" as used herein refers to all proteins present in the periplasmic space between the outer membrane and inner membrane of prokaryotes. According to one embodiment of the present invention, the periplasmic protein may be membrane-associated proteins that directly or indirectly bind to the outer or inner membrane. The present inventors, having conceived that in prokaryotes consisting of a double membrane structure of inner and outer membranes, proteins in the intermembrane space can be eluted with only minimal physical and chemical stimulation sufficient to expose the periplasmic space by disintegrating only the outer membrane rather than dissolving the entire cell membrane, confirmed that intact periplasmic proteins can be eluted without damage by adding an organic solvent such as lower alcohol or nitrile.

The term "protein elution" as used herein refers to the process of extracting proteins of prokaryotes comprised in a biological sample into an external environment outside the cell wall. Therefore, the terms "protein elution" are used synonymously with "protein extraction" and "protein isolation".

The term "alkyl" as used herein refers to saturated hydrocarbon groups of straight or branched chain, and comprises, for example, methyl, ethyl, propyl, isopropyl, and the like. Ci-C₃ alkyl refers to an alkyl group with an alkyl unit of 1 to 3 carbon atoms. When C₁-C₃ alkyl is substituted, the number of carbon atoms of the substituent is not comprised.

According to a concrete embodiment, the alcohol is selected from the group consisting of methanol, ethanol, 1-propanol, 2-propanol, 1-butanol and 2-butanol.

According to a concrete embodiment, the R-CN is C₁-CN (acetonitrile).

According to a concrete embodiment of the present invention, the organic solvent used in the present invention is 1-propanol. More concretely it is 20-60 v/v% 1-propanol, and most concretely 30-60 v/v% 1-propanol.

According to a concrete embodiment, the organic solvent used in the present invention is 1-butanol. More concretely it is 3-12 v/v% 1-butanol, and most concretely 5-10 v/v% 1-butanol.

According to a concrete embodiment, the organic solvent used in the present invention has an octanol-water partition coefficient (Kow) of 0.3 - 1.1, more concretely a Kow of 0.4 - 1, and most concretely a Kow of 0.5 - 0.9. The terms "octanol-water partition coefficient" as use herein refer to the relative distribution ratio of the administered sample between the water phase and the oil phase, which are immiscible solvents, and indicates hydrophobicity if greater than 1 and hydrophilicity if less than 1.

The terms "biological sample" as used herein refer to any substance that may contain the target protein, or cells or microorganisms expressing the target protein, or cultures thereof. The terms are used as a generic term for materials taken from samples isolated from living organisms (blood, plasma, urine, saliva, tissues, organs, etc.) or from the environment (such as water, air, soil, etc.), or materials artificially mixed.

In another aspect of the present invention, there is provided a method for eluting a protein from a prokaryote comprising adding a hypertonic solution or a hypotonic solution to a biological sample comprising the prokaryote.

Since the prokaryotes from which proteins are to be isolated and biological samples containing them have already been described above in detail, descriptions thereof are omitted to avoid excessive redundancy.

According to the present invention, when an osmotic stimulus is applied by adding a hypertonic or hypotonic solution to a sample comprising prokaryotes, the cell wall of the prokaryote is efficiently dissolved by osmotic lysis, thereby providing undamaged prokaryote-derived proteins, and analysis of the obtained protein can be initiated without additional purification process.

The term "osmotic lysis" as used herein refers to the process of dissolving cells by allowing excess water to flow out of or diffuse into the cells through osmotic imbalance caused by adding a hypertonic solution or a hypotonic solution to the cells. Various solutes may be used without limitation for the hypertonic solution as long as the solution has a high concentration enough for induce significant difference in concentration from that of the cells or their culture medium so that water sufficient for cell lysis can flow out. A low-concentration solution with a significant difference in concentration from that of the cells or their culture medium so that water sufficient for cell lysis can flow in may be used without limitation as the hypotonic solution, such as distilled water.

According to a concrete embodiment, the method of the present invention is performed by adding a hypertonic solution to the biological sample.

More concretely, the hypertonic solution comprises at least one solute selected from the group consisting of glucose, sucrose, mannitol, trehalose, sorbitol, potassium chloride (KCl) and sodium chloride (NaCl).

More concretely, the hypertonic solution is 100 mM - 5 M sodium chloride (NaCl) solution, even more concretely 200 mM - 3M NaCl solution, and most concretely 400 mM - 1.5 M NaCl solution.

In another aspect of the present invention, there is provided a method for detecting a protein of a prokaryote in a biological sample comprising:
(a) eluting the protein of the prokaryote using the method of any one of claims 1 to 10; and
(b) performing mass spectrometry on the eluted protein.

According to a concrete embodiment, the step (b) is carried out by a mass spectrometry method selected from the group consisting of Matrix-Assisted Laser Desorption/Ionization Time of Flight mass spectrometry (MALDI-TOF), Surface Enhanced Laser Desorption/Ionization Time of Flight mass spectrometry (SELDI-TOF), Electrospray Ionization Time of Flight mass spectrometry (ESI-TOF), Liquid Chromatography-Mass Spectrometry (LC-MS), and Liquid Chromatography-Mass Spectrometry/Mass Spectrometry (LC-MS/MS).

More concretely, the step (b) is performed using MALDI-TOF mass spectrometry.

MALDI-TOF mass spectrometry is a method in which a sample supported by a matrix is desorbed and ionized by irradiation with a laser, and then the molecular weights of the generated ions are analyzed by measuring the time (Time-of-Flight) taken for the ions to reach a detector. According to this method, it is possible to quickly and accurately measure the mass of a large biomolecule such as a protein, because fragmentation of the target material does not occur.

According to the present invention, the protein elution method applying organic solvents or osmotic stimulation can be directly used for analysis without a additional desalting process since the final background product does not contain salt that may affect the spectrum during mass spectrometry, and shows remarkably outstanding reliability and sensitivity for mass spectrometry of periplasmic space protein or membrane-associated proteins of gram-negative bacteria compared to conventional ultrasonic, surfactant, urea, and OG treatment methods.

In another aspect of the present invention, there is provided a composition for eluting a protein of a prokaryote comprising, as an active ingredient, at least one organic solvent selected from the group consisting of C₁-C₄ alcohol and R-CN (wherein R is a straight or branched C₁-C₃ alkyl).

In another aspect of the present invention, there is provided a composition for eluting a protein of a prokaryote comprising, as an active ingredient, a hypertonic solution or a hypotonic solution.

Since the organic solvent, hypertonic and hypotonic solutions used in the present invention, and the prokaryotes from which the proteins are to be isolated have already been described in detail, descriptions thereof are omitted to avoid excessive redundancy.

### EFFECTS OF THE INVENTION

The features and advantages of the present invention are summarized as follows:
(a) The present invention provides a method for eluting proteins from prokaryotes.
(b) The present invention enables easy break down of bacterial cell walls to obtain intact proteins without damage by a simple process of adding organic solvents including lower alcohols or nitrile derivatives; or applying osmotic stimulation to samples containing pathogenic bacteria or the like.
(c) The present invention may be usefully applied to rapid and accurate identification of periplasmic proteins of gram-negative bacteria without additional purification process.

### BRIEF DESCRIPTION OF THE DRAWINGS

Figure 1a represents the comparison between the results of elution of gram-negative bacteria-derived proteins using the osmotic pressure method of the present invention and the conventional isolation method (ultrasonic, sodium dodecyl sulfate (SDS), urea, and Octyl β-glucoside (OG) treatment methods) by SDS-PAGE analysis. Figure 1b shows a diagram of protein extraction results at each concentration of sodium chloride used in the osmotic pressure method.
Figure 2 represents the results of SDS-PAGE analysis for various gram-negative bacteria-derived proteins eluted using the osmotic pressure method of the present invention.
Figure 3 represents the results of SDS-PAGE analysis for each gram-negative bacteria-derived protein eluted using 1-propanol (Figs. 3a and 3d), acetonitrile, methanol, 2-propanol, ethanol, 1-butanol and 2-butanol (Figs. 3b and 3c).
Figure 4 represents the results of MALDI-TOF analysis for KPC-2 and CTX-M-1 proteins eluted by the method of the present invention.
Figure 5 shows the results of high-resolution mass spectrometry for KPC-2 intact proteins eluted by the method of the present invention represented by Q Exactive HF-X (Fig. 5a) and Q-TOF (Fig. 5b).
Figure 6 represents the results of high-resolution mass spectrometry (Q Exactive HF-X) for CTX-M-1 intact proteins eluted by the method of the present invention.

### DETAILED DESCRIPTION OF THE INVENTION

Hereinafter, the present invention will be described in further detail by examples. It would be obvious to those skilled in the art that these examples are intended to be more concretely illustrative and that the scope of the present invention as set forth in the appended claims is not limited to or by the examples.

### Examples

### Example 1: Pretreatment for Elution of Expressed Proteins

### Cell Culture

*E.coli* transformed with plasmids comprising KPC-2, CTX-M-1, and NDM-1 genes were inoculated into Luria-bertani liquid medium containing 50µg/ml ampicillin antibiotic, and then cultured at 37 °C for more than 16 hours.

### Cell Lysis

### Sample preparation by osmotic gradient

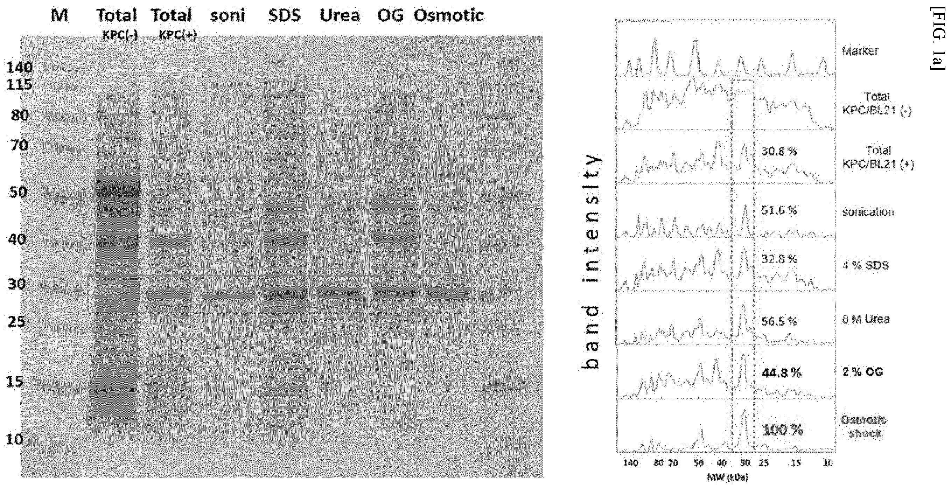

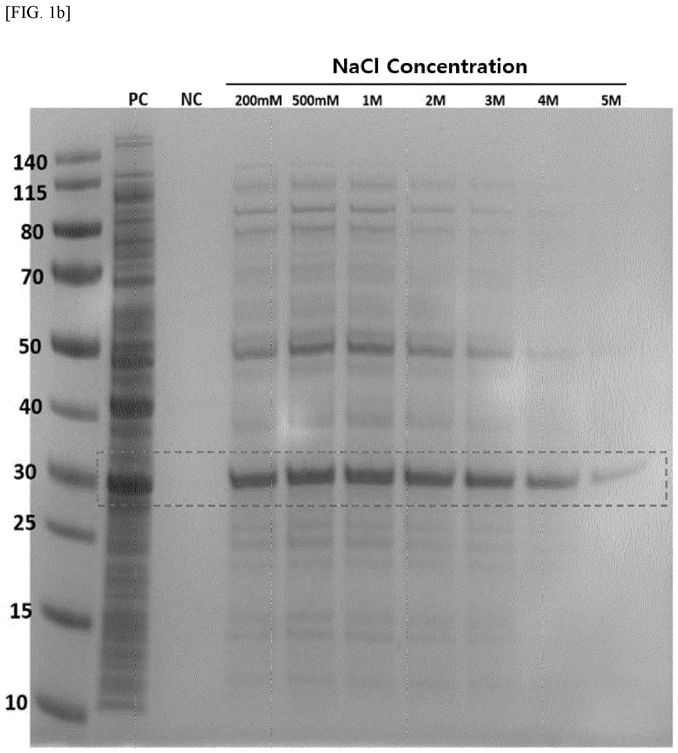

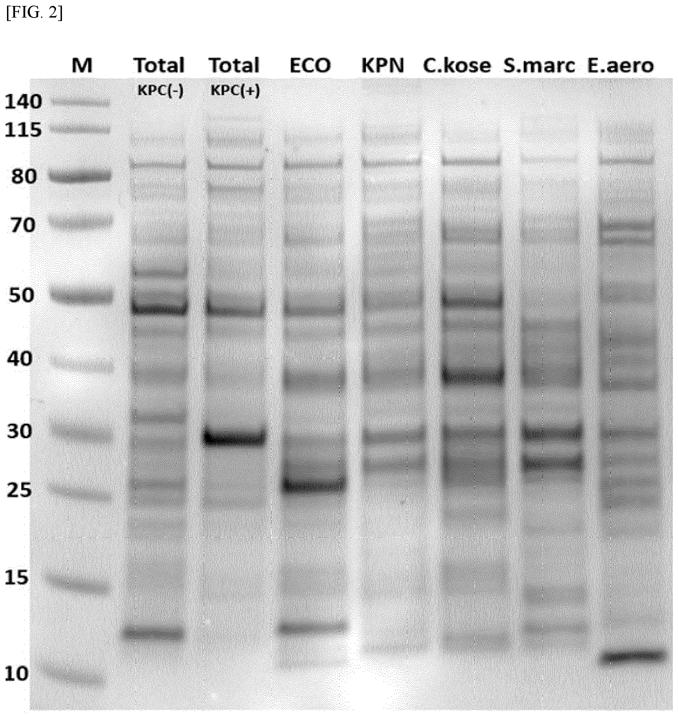

For sample pretreatment, culture medium for the strain expressing the KPC-2 protein was centrifuged at 4,000 rpm for 15 minutes, and the supernatant was removed to harvest the cells. The harvested cells were resuspended with hypertonic solution (500 mM NaCl, 25mM Tris-HCl, pH 8.0). The suspension was reacted at room temperature for 10 minutes. Thereafter, the supernatant was removed via centrifugation at 14,000 g at 4°C for 10 minutes. The remaining cells were resuspended in tertiary distilled water and reacted at room temperature for 10 minutes. Then, the supernatant was stored after centrifugation at 14,000 g at 4°C for 10 minutes. The efficiency of the osmotic pressure method was confirmed by comparing with conventional protein extraction methods by SDS-PAGE analysis (FIG. 1). In addition, various gram-negative bacteria such as *Escherichia coli, Klebsiella pneumonia, Citrobacter koseri, Staphylococcus aureus* and *Enterobacter aerogenes* were eluted by the osmotic pressure method, confirming effective extraction of the proteins (FIG. 2).

### Sample pretreatement by organic solvent

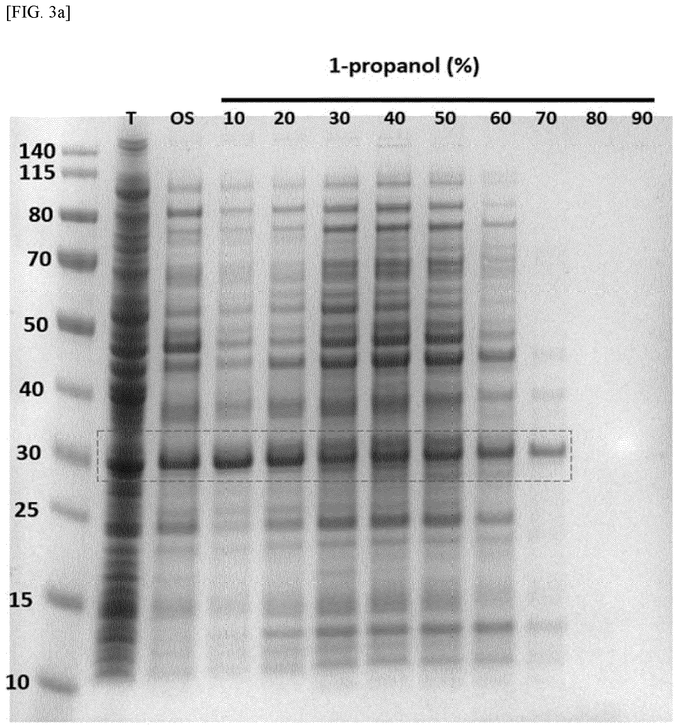

For sample pretreatment, culture medium for the strain expressing the CTX-M-1 and NDM-1 proteins were centrifuged at 4,000 rpm for 15 minutes, and the supernatant was removed to harvest the cells. Harvested strains expressing CTX-M-1 were treated with 10 to 90% 1-propanol and allowed to react at room temperature for 10 minutes. Then, the supernatant was collected by centrifugation at 14,000 g at 4°C for 10 minutes. The size of the eluted proteins was confirmed through SDS-PAGE analysis of the supernatant sample (FIG. 3a). 10 to 70% of organic solvent was used for mass spectrometry.

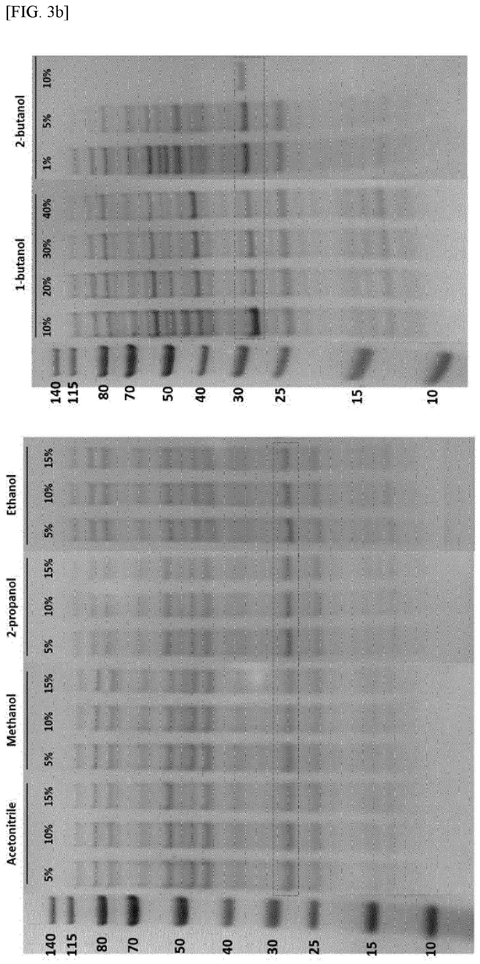

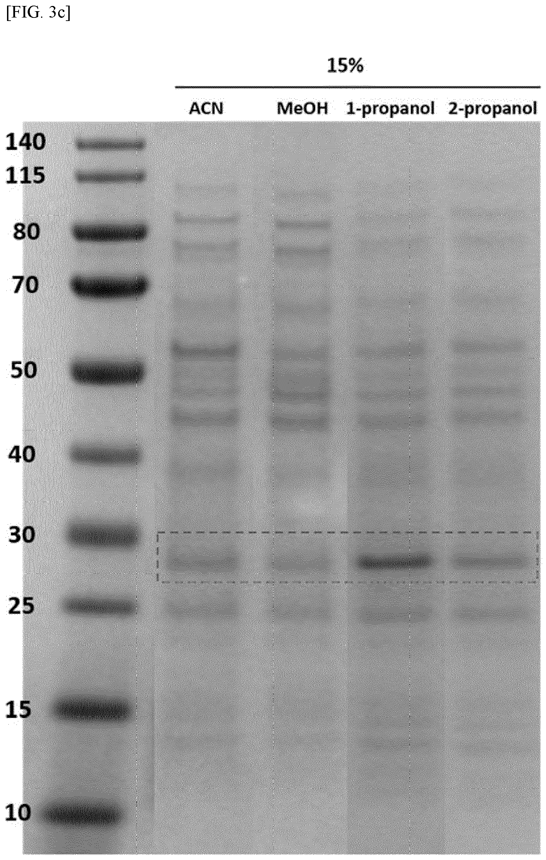

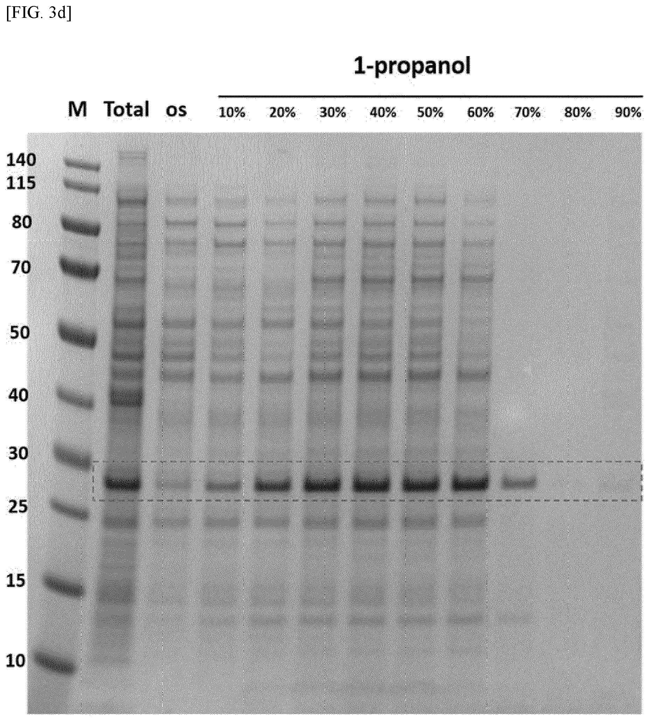

Furthermore, additional organic solvents including acetonitrile, methanol, 2-propanol, ethanol, 1-butanol, and 2-butanol were pretreated in the same way to the NDM-1-expressing strain, and the eluted proteins were confirmed (FIG. 3b). In the case of the NDM-1 protein, which belongs to bacterial membrane-bound proteins, it could be selectively separated using organic solvents. It was possible to elute membrane-bound proteins according to the characteristics of the organic solvents through the octanol-water partition coefficient, which is widely used to determine the hydrophilicity and hydrophobicity of solutes. Among the organic solvents tested, 1-propanol had the best elution effect (FIG. 3c), and the 30-60% conditions showed high elution efficiency among various concentrations that were applied (FIG. 3d).

### Example 2: Confirmation of Intact Protein by Mass Spectrometry

### Intact protein analysis using low-resolution mass spectrometry

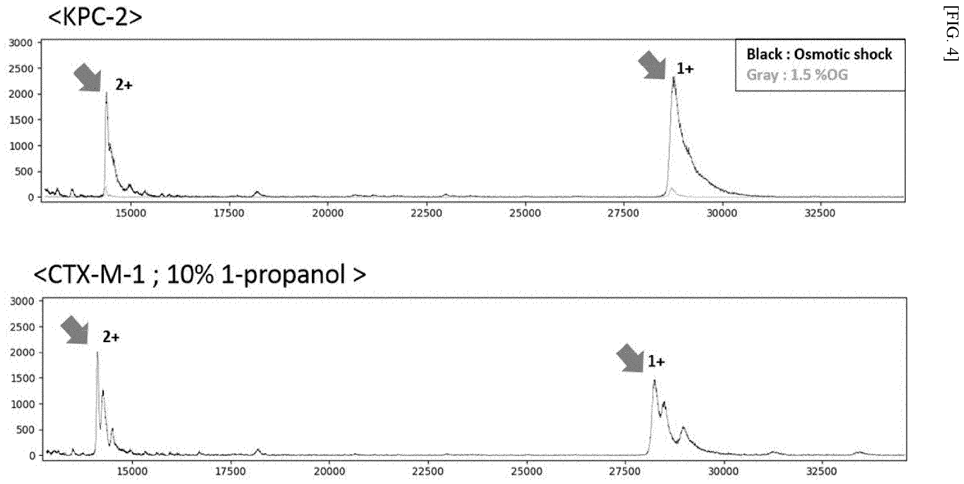

A mass spectrometry spectrum of the KPC-2 protein was obtained using a Bruker Biotyper Smart LT MALDI-TOF MS instrument. First, 1µl of the supernatant obtained from the osmotic method was spotted and dried on a MALDI plate, then 1µl of matrix SA (20 mg/mL in 0.1% TFA/50% acetonitrile) was spotted, covered and dried completely to perform MALDI-TOF analysis. The pulse ion extraction time was 120 ns. The spectrum was measured in linear mode in ion sources 1 and 2, using accelerating voltages of 18 kV and 16.29 kV, and a lens voltage of 5.4 kV. Laser power was set to 75% and a total of 200 laser shots were irradiated by 40 shots, and each spectral data was accumulated. A mass spectrometry spectrum of the expressed KPC-2 intact protein in the range of 12,600 to 36,000 m/z was obtained, and the +1 and +2 valent proteins were also simultaneously detected. To verify the dissolution effect of the osmotic pressure method, the same amount of sample was lysed using n-octyl-b-D-glucopyranoside (OG), a nonionic surfactant to dissolve the strain, and then MALDI-TOF analysis was performed. As a result, the osmotic elution method provided highly purified data values with an increase in strength about 10 times (FIG. 4). In addition, after dissolving the strain expressing the CTX-M-1 protein with 1-propanol, MALDI-TOF analysis was performed using the same method with 10% fraction, and the spectral results for the eluted intact CTX-M-1 protein were obtained (FIG. 4).

### Intact protein analysis using high-resolution mass spectrometry

In order to confirm the sequence and range of the intact protein expressed within the strain, liquid chromatography and high resolution mass spectrometry were performed using Thermo Q Exactive HF-X and Agilent Q-TOF mass spectrometer system. The protein concentration of the sample pretreated with the osmotic concentration gradient was measured at a wavelength of 280 nm using a nano-drop quantitative analyzer, and 50 ng and 200 ng of protein were injected into the column for Q Exactive analysis and Q-TOF analysis, respectively. Samples pretreated with an organic solvent were dried using a speed-vac device, and its protein concentration was measured at a wavelength of 280 nm using a nano-drop quantitative analyzer after dissolving well in 0.1% formic acid. The same amount used for the osmotic method was injected into the column.

### (1) QExactive HF-X analysis

Protein samples were separated using nano liquid chromatography with a PLRP-S column (100 µm × 600 mm), and the gradient conditions for loading and separation of the samples were as follows:
- Buffer A : 0.1% formic acid dissolved in water; Buffer B : 0.1% formic acid dissolved in acetonitrile
- Sample loading : 0-5 min, 5% (B), flow rate 4 µL/min
- Separation gradient:
   5.0-5.1 min, 5% -> 20% (B), flow rate 1000 nL/min
   5.1-14 min, 20% -> 95% (B), flow rate 1000 nL/min
   14-15.5 min, 95% (B), flow rate 1000 nL/min
   15.5-16 min, 95% -> 5% (B), flow rate 1000 nL/min
   16-20 min, 5% (B), flow rate 1000 nL/min
- Mass spectrometry parameters
   Resolution : full length MS 240,000, MS2 120,000
   Range : full length MS MS 800 - 1,100 m/z or 1,300 - 2,000 m/z, 1500 ms
   MS2 : 1500 ms, NCE (HCD) 35, 1-8 valent ionized substances are excluded from MS2
   AGC : 1e6

### (2) Q-TOF analysis

Protein samples were separated using micro liquid chromatography with a C18 column (2.1 × 150mm), and the gradient conditions used for separation were as follows:
- Buffer A : 0.1% formic acid dissolved in water; Buffer B : 0.1% formic acid dissolved in acetonitrile
- Separation gradient:
   0-1 min, 5% -> 10% (B), flow rate 200 µL/min
   1-25 min, 10% -> 80% (B), flow rate 200 µL/min
   25-27 min, 80% (B), flow rate 200 µL/min
   27-28 min, 80% -> 5% (B), flow rate 200 µL/min
   28-30 min, 5% (B), flow rate 200 µL/min
- Mass spectrometry parameters
   Gas Temp 320°C, Drying Gas 8 1/min, Nebulizer 35 psi, Sheath Gas Temp 350 °C, Sheath Gas Flow 11 1/min
   Range: full length MS: 1,300 - 2,000 m/z
   Acquisition rate: 3 spectra/s, Time 333.3 ms/spectrum

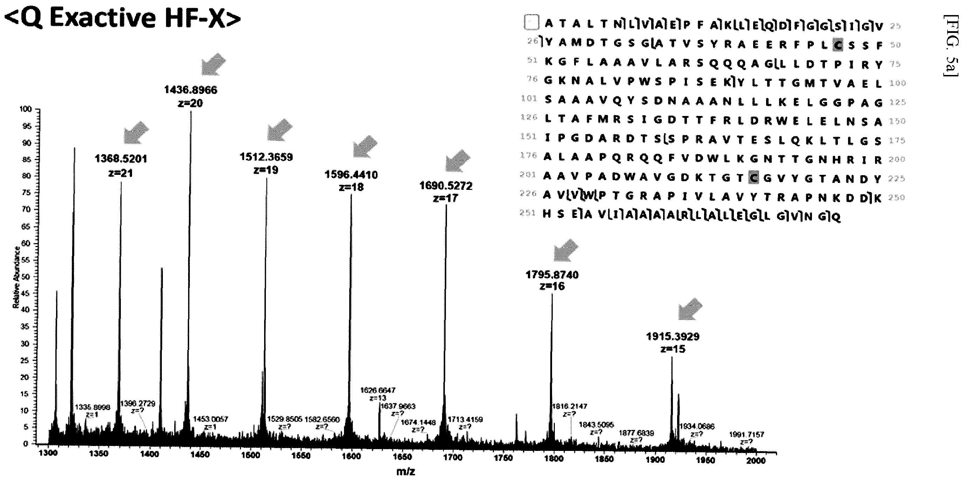

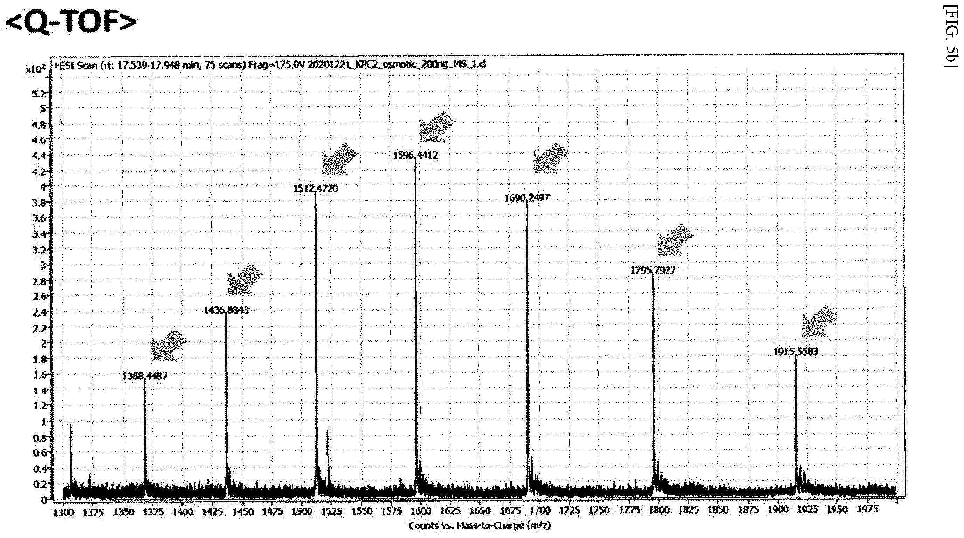

The software utilized for identifying intact proteins by Q Exactive HF-X was the "Prosight Light" program developed by Northwestern University of Illinois, USA. The MS1 and MS2 results of the intact protein mass spectrometry were matched with the protein sequences of KPC-2 and CTX-M-1. In the case of KPC-2 protein, MS1 matched with a difference of 4.68 ppm, and in the case of MS2, 51 fragments matched (FIG. 5a). CTX-M-1 matched with MS1 with a difference of -32.83 ppm, and 38 fragments were matched with MS2 (FIG. 5b). The KPC-2 protein was identified as an active form (AA 22-293) in which the signal peptide at residue portions 1 to 21 was truncated, and a disulfide bond was present at cysteines of positions 68 and 237. CTX-M-1 was identified as a truncated active form in which signal peptide of 1 to 28 residues was removed (AA 29-291).

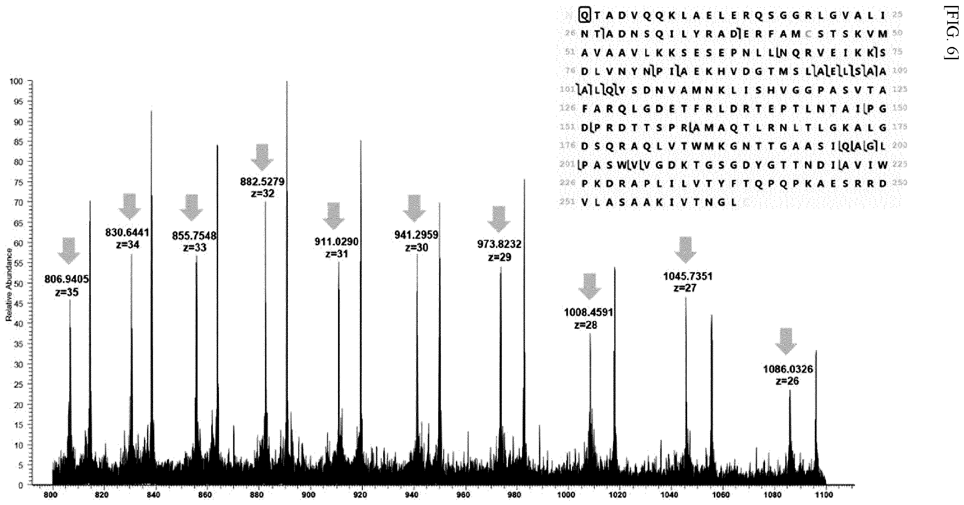

Regarding Q-TOF spectrometry, the MS1 spectrum of KPC-2 intact protein was confirmed and the same m/z value as Q Exactive HF-X was obtained (FIG. 6).

Having described specific embodiment of the present invention in detail above, it is to be understood that variants and modifications thereof falling within the spirit of the invention may become apparent to those skilled in this art, and the scope of this invention is to be determined by appended claims and their equivalents.

## Claims

1. A method for eluting a protein of a prokaryote comprising adding at least one organic solvent selected from the group consisting of C₁-C₄ alcohols and R-CN (wherein R is a straight or branched C₁-C₃ alkyl) to a biological sample comprising the prokaryote.

2. The method of claim 1, wherein the alcohol is selected from the group consisting of methanol, ethanol, 1-propanol, 2-propanol, 1-butanol and 2-butanol.

3. The method of claim 1, wherein the R-CN is acetonitrile.

4. The method of claim 1, wherein the prokaryote is gram-negative bacteria.

5. The method of claim 4, wherein the gram-negative bacteria is selected from the group consisting of *Escherichia coli, Citrobacter koseri, Enterobacter aerogenes, Escherichia albertii, Escherichia blattae, Escherichia fergusonii, Escherichia hermannii, Escherichia vulneris, Acinetobacter baumannii, Acinetobacter junii, Acinetobacter boissieri, Acinetobacter calcoaceticus, Acinetobacter haemolyticus, Acinetobacter nosocomialis, Acinetobacter schindleri, Acinetobacter ursingii, Klebsiella pneumoniae, Staphylococcus aureus, Streptococcus pneumoniae, Neisseria gonorrhoeae, Pseudomonas aeruginosa, Samonella typhimurium, Clostridium difficile, Shigella, Yersinia pestis, Haemophilus, Brucella, Legionella, Burkholderia cepacia, Vibrio* and *Stenotrophomonas maltophilia.*

6. A method for eluting a protein from a prokaryote comprising adding a hypertonic solution or a hypotonic solution to a biological sample comprising the prokaryote.

7. The method of claim 6, wherein the method comprises adding hypertonic solution to the biological sample.

8. The method of claim 7, wherein the hypertonic solution comprises at least one solute selected from the group consisting of glucose, sucrose, mannitol, trehalose, sorbitol, potassium chloride (KCI) and sodium chloride (NaCl).

9. The method of claim 8, wherein the hypertonic solution is a 100 mM- 5M sodium chloride (NaCl) solution.

10. The method of claim 6, wherein the prokaryote is gram-negative bacteria.

11. A method for detecting a protein of a prokaryote in a biological sample comprising:
(a) eluting the protein of the prokaryote using the method of any one of claims 1 to 10; and
(b) performing mass spectrometry on the eluted protein.

12. The method of claim 11, wherein the step (b) is carried out by a mass spectrometry method selected from the group consisting of Matrix-Assisted Laser Desorption/Ionization Time of Flight mass spectrometry (MALDI-TOF), Surface Enhanced Laser Desorption/Ionization Time of Flight mass spectrometry (SELDI-TOF), Electrospray Ionization Time of Flight mass spectrometry (ESI-TOF), Liquid Chromatography-Mass Spectrometry (LC-MS), and Liquid Chromatography-Mass Spectrometry/Mass Spectrometry (LC-MS/MS).

13. The method of claim 12, wherein the step (b) is carried out by MALDI-TOF (Matrix-Assisted Laser Desorption/Ionization Time of Flight) mass spectrometry.

14. A composition for eluting a protein of a prokaryote comprising, as an active ingredient, at least one organic solvent selected from the group consisting of C₁-C₄ alcohol and R-CN (wherein R is a straight or branched C₁-C₃ alkyl).

15. A composition for eluting a protein of a prokaryote comprising, as an active ingredient, a hypertonic solution or a hypotonic solution.
